# EUROPÄISCHE PATENTANMELDUNG

(11) **EP 1 982 666 A1**
(43) Veröffentlichungstag der Anmeldung: **22.10.2008**
(21) Anmeldenummer: 07007972.8
(22) Anmeldetag: 19.04.2007
(51) Int. Cl.: A61B 19/00, A61B 17/00

(54) **Vorrichtung zur Erfassung der räumlichen Position**

(71) Anmelder: Weber Instrumente GmbH, 78576 Emmingen-Liptingen (DE)
(72) Erfinder: Weber, Helmut, 78576 Emmingen-Liptingen (DE)
(74) Vertreter: Modrow, Stephanie

(57) **Zusammenfassung**

Die Erfindung betrifft eine Vorrichtung (20, 70, 120) zur Erfassung der räumlichen Position, welche über eine Befestigungsvorrichtung (30, 80, 130) an einem chirurgischen Instrument (10, 60, 100), welches eine Längsachse (l₁₀, l₆₀, l₁₀₀) aufweist, anbringbar ist, wobei die Befestigungsvorrichtung (30, 80, 130) eine Durchgangsöffnung (33, 83, 133) aufweist, welche von dem Instrument (10, 60, 100) durchsetzt wird, und dass die Befestigungsvorrichtung (30, 80, 130) um die Längsachse (l₁₀, l₆₀, l₁₀₀) des Instruments (10, 60, 100) drehbar gelagert ist

## Beschreibung

Die Erfindung betrifft eine Vorrichtung zur Erfassung der räumlichen Position gemäß dem Oberbegriff des Patentanspruchs 1.

Bekannt sind in der Chirurgie Vorrichtungen zur Erfassung der räumlichen Position, mit welchen chirurgische Eingriffe überwacht werden können. Derartige Vorrichtungen zur Erfassung der räumlichen Position werden an den verwendeten chirurgischen Instrumenten angeordnet, um diese zu überwachen, und werden daher auch als Tracker bezeichnet. Bekannt sind dabei sowohl aktive als auch passive Tracker.

Die passiven Tracker weisen Reflektoren auf, welche Licht, welches von im Operationsraum angeordneten Lichtsendern ausgesandt wird, reflektieren, wobei das an den Reflektoren reflektierte Licht beispielsweise mit CCD-Kameras aufgenommen wird und anhand der ermittelten Lichtflecken die Position und Orientierung der Reflektoren und somit des Instruments relativ im Raum berechnet wird. Alternativ weisen die sogenannten aktiven Tracker selbst entsprechende Lichtquellen, insbesondere Infrarot-LEDs, auf, wobei das von den aktiven Trackern imitierte Licht von entsprechenden im Operationsraum angeordneten Überwachungskameras detektiert wird, woraus wiederum die Position und Orientierung im Raum bestimmt werden kann.

Wesentlich dabei ist, dass die Vorrichtungen zur Erfassung der räumlichen Position in einer definierten relativen Lage zu dem Instrument angeordnet sind. Daher ist es bekannt, die Vorrichtungen zur Erfassung der räumlichen Position über eine Befestigungsvorrichtung an dem chirurgischen Instrument anzuordnen. Diese Befestigungsvorrichtungen sind beispielsweise als Schraubklemme ausgebildet, so dass die Befestigungsvorrichtung fest an das Instrument angeschraubt werden kann. Bei Bewegung des chirurgischen Instruments führt somit auch die Vorrichtung zur Erfassung der räumlichen Position die entsprechenden Bewegungen aus.

Bei der Operation ist jedoch darauf zu achten, dass die Vorrichtung zur Erfassung der räumlichen Position immer im Blickwinkel der Überwachungskameras ist. Ansonsten besteht die Gefahr, dass die mit dem chirurgischen Instrument durchgeführten Bewegungen nicht erfasst werden und somit Verletzungsgefahr für den Patienten besteht.

Bei der Verwendung vieler chirurgischer Instrumente ist jedoch eine Drehung der chirurgischen Instrumente um ihre Längsachse von Nöten. Bei Verwendung der bekannten Vorrichtungen zur Erfassung der räumlichen Position kann das Instrument jedoch nur um wenige Winkelgrade um seine Längsachse in eine Richtung gedreht werden und muss anschließend um wenige Winkelgrade in die entgegengesetzte Richtung gedreht werden, damit die Vorrichtung zur Erfassung der räumlichen Position ständig im Blickwinkel der Überwachungskameras ist und nicht beispielsweise bei Drehung um etwa 180 Grad um die Längsachse unterhalb des Instruments und somit außerhalb des Blickwinkels der Überwachungskameras angeordnet ist. Dies verkompliziert in der Regel die durchzuführende Operation.

Die Aufgabe der Erfindung besteht daher darin, eine Vorrichtung zur Erfassung der räumlichen Position bereit zu stellen, welche bei der Operation einfacher zu handhaben ist.

Die Aufgabe wird gelöst durch eine Vorrichtung zur Erfassung der räumlichen Position mit den Merkmalen des Patentanspruchs 1.

Vorteilhafte Ausgestaltungen und Weiterbildungen sind in den Unteransprüchen angegeben.

Die Erfindung beruht auf der Erkenntnis, dass eine drehbare Lagerung der Befestigungsvorrichtung um die Längsachse des Instruments die genannten Nachteile beseitigt. Die erfindungsgemäße Befestigungsvorrichtung weist daher eine Durchgangsöffnung auf, welche von dem chirurgischen Instrument durchsetzt wird, wobei die Befestigungsvorrichtung um die Längsachse des Instruments drehbar gelagert ist. Dadurch wird ermöglicht, mit einer Hand das chirurgische Instrument beliebig um die Längsachse zu drehen, während mit der anderen Hand die Befestigungsvorrichtung derart gehalten wird, dass sich die Vorrichtung zur Erfassung der räumlichen Position immer oberhalb des chirurgischen Instruments im Blickwinkel der Überwachungskamera im Operationssaal befindet. Die Handhabung des chirurgischen Instruments mit der Vorrichtung zur Erfassung der räumlichen Position wird somit deutlich vereinfacht, da das chirurgische Instrument beliebig um seine Längsachse gedreht werden kann.

Bei einer besonders bevorzugten Ausführungsform der Erfindung ist die Befestigungsvorrichtung in axialer und/oder radialer Richtung fixierbar. Wesentlich ist, dass die Vorrichtung zur Erfassung der räumlichen Position in einer bekannten relativen Lage zu dem chirurgischen Instrument angeordnet ist, da die relative Lage des chirurgischen Instruments im Hinblick auf den Patienten berechnet wird aus Signalen, die die Vorrichtung zur Erfassung der räumlichen Position abgibt. Falls somit das chirurgische Instrument seine relative Lage zu der Vorrichtung zur Erfassung der räumlichen Position verändert, kann dies in einer Verletzungsgefahr für den Patienten resultieren. Insbesondere ist eine Fixierung in axialer Richtung von höchster Bedeutung, da ansonsten das chirurgische Instrument durch die Durchgangsöffnung der Befestigungsvorrichtung beispielsweise zu weit in den Patienten eingeführt wird und dort lebensgefährliche Verletzungen hervorrufen könnte.

Vorzugsweise sind zur axialen und/oder radialen Fixierung eine umlaufende Nut, ein umlaufender Vorsprung und/oder ein umlaufender Anschlag vorgesehen.

Bei einer vorteilhaften Weiterbildung kann zur axialen und/oder radialen Fixierung auch wenigstens eine in der Durchgangsöffnung der Befestigungsvorrichtung angeordnete Lagerbuchse, insbesondere eine Kunststofflagerbuchse, vorgesehen werden.

Vorzugsweise ist zur axialen und/oder radialen Fixierung ein Kugellager vorgesehen, welches zudem eine besonders leicht drehbare Lagerung der Befestigungsvorrichtung auf dem chirurgischen Instrument ermöglicht.

Bei einer besonders bevorzugten Ausführungsform der Erfindung sind zur axialen und radialen Fixierung zwei Schrägkugellager vorgesehen. Diese ermöglichen insbesondere eine spielfreie Fixierung sowohl in axialer als auch in radialer Richtung, wodurch die Genauigkeit der Erfassung der räumlichen Position gewährleistet wird.

Zur axialen Fixierung kann in einer bevorzugten Ausführungsform eine Feder vorgesehen werden, welche die Befestigungsvorrichtung gegen einen an dem chirurgischen Instrument angeordneten Anschlag presst. Durch diese Federbeaufschlagung wird eine spielfreie Fixierung in axialer Richtung erreicht.

In einer bevorzugten Ausführungsform ist zur axialen Fixierung eine Mutter mit einem Gewinde vorgesehen, welche in ein an dem Instrument angeordnetes Gewinde eingreift. Beim Anziehen der Mutter wird die Befestigungsvorrichtung gegen einen an dem Instrument angeordneten Anschlag gepresst. Durch die Steigung des Gewindes ist auch hier eine spielfreie axiale Fixierung der Befestigungsvorrichtung an dem chirurgischen Instrument möglich.

Um zu verhindern, dass sich die axiale Fixierung wieder löst, indem beispielsweise bei der Handhabung des chirurgischen Instruments sich die Mutter löst, ist bei einer bevorzugten Ausführungsform die Mutter in der gewünschten Position fixierbar, beispielsweise mittels Laserschweißen, Verkleben oder einer mechanischen Arretiervorrichtung.

Vorzugsweise ist die Vorrichtung zur Erfassung der räumlichen Position als aktiver oder als passiver Tracker ausgebildet. Vorzugsweise ist die Vorrichtung zur Erfassung der räumlichen Position als optischer Tracker ausgebildet oder weist ein GPS-System auf, um die erforderliche Genauigkeit der Positionsbestimmung zu ermöglichen.

Die Erfindung wird anhand der folgenden Figuren ausführliche erläutert. Es zeigt
- Fig. 1a: eine Seitenansicht eines ersten Ausführungsbeispiels der Erfindung,
- Fig. 1b: einen Axialschnitt durch einen Ausschnitt des Ausführungsbeispiels gemäß Fig. 1a,
- Fig. 2a: eine Seitenansicht eines zweiten Ausführungsbeispiels gemäß der Erfindung,
- Fig. 2b: eine Axialschnitt durch einen Ausschnitt des Ausführungsbeispiels gemäß Fig. 2a,
- Fig. 2c: einen Ausschnitt einer Draufsicht auf das Instrument gemäß Fig. 2a,
- Fig. 3a: eine Seitenansicht eines dritten Ausführungsbeispiels gemäß der Erfindung,
- Fig. 3b: eine Frontansicht auf das Ausführungsbeispiel gemäß Fig. 3a,
- Fig. 3c: eine Draufsicht auf das Ausführungsbeispiel gemäß Fig. 3a und
- Fig. 3d: einen Längsschnitt durch einen Teil des Ausführungsbeispiels gemäß Fig. 3a.

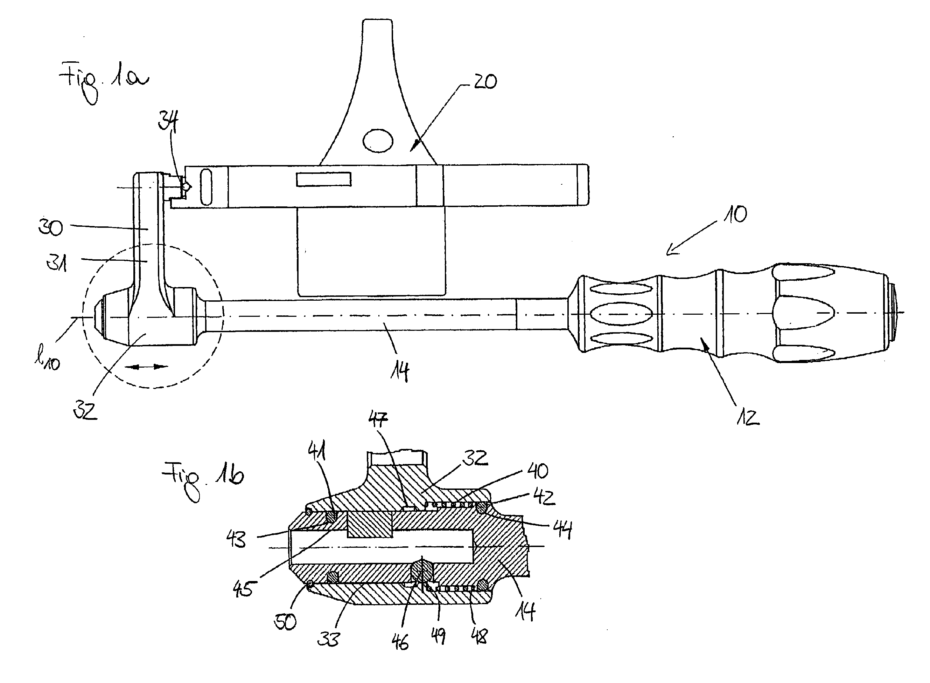

In den Figuren 1a und 1b ist ein erstes Ausführungsbeispiel der Erfindung dargestellt. Fig. 1a zeigt ein chirurgisches Instrument 10 mit einem Handgriff 12, an welchem ein Schaft 14 angeordnet ist. Die Längsachse des Schafts 14 bildet eine Längsachse l₁₀ des chirurgischen Instruments 10.

Das distale Ende des Schafts 14 weist eine Längsbohrung 45 auf, in welche ein beliebiges Arbeitsende eines chirurgischen Instruments einsetzbar ist. Um dieses Arbeitsende in der Längsbohrung 45 fixieren zu können, ist um das distale Ende des Schafts 14 eine Hülse 32 angeordnet, welche entlang der Längsachse I₁₀ verschiebbar ist. Die Hülse 32 weist eine Durchgangsöffnung 33 auf, welche von dem Schaft 14 des chirurgischen Instruments 10 durchsetzt wird. Auf der Innenseite der Hülse 32 ist eine umlaufende Nut 47 angeordnet, während in der Wandung der Längsbohrung 45 eine Durchgangsbohrung angeordnet ist, in welcher eine Sicherungskugel 46 angeordnet ist. Wird die Hülse 32 derart in Längsrichtung verschoben, dass die Nut 47 axial auf gleicher Höhe mit der Sicherungskugel 46 zu liegen kommt, kann die Sicherungskugel 46 in die Nut 47 radial nach außen ausweichen und gibt so die Möglichkeit, in die Längsbohrung 45 das gewünschte Arbeitsende einzuschieben. Wird die Hülse 32 in Längsrichtung wieder derart verschoben, dass die Nut 47 axial beabstandet zu der Sicherungskugel 46 ist, wird die Sicherungskugel 46 geringfügig in die Längsbohrung 45 hineingedrückt und greift dort in eine entsprechende Ausnehmung des eingesetzten Arbeitsendes ein, um dieses sicher in der Längsbohrung 45 zu halten. Auf diese Weise wird eine Kupplung für ein auswechselbares Arbeitsende gebildet.

Die gewünschte Verschiebung der Hülse 32 zur Kupplung wird durch eine Feder 40 erreicht. Diese ist als Schraubenfeder ausgebildet und liegt auf der Außenseite des Schafts 14 an, wobei sie zwischen einem an dem Schaft 14 umlaufend angeordneten Vorsprung 48 und einem auf der Innenseite der Hülse 32 umlaufend angeordneten Vorsprung 49 zuliegen kommt. Die Feder 40 ist derart bemessen, dass aufgrund der Federkraft die Hülse 32 derart entlang der Längsrichtung des Schafts 14 verschoben wird, dass die Nut 47 in distaler Richtung axial beabstandet vor der Sicherungskugel 46 zuliegen kommt und zudem die Hülse 32 gegen einen Wellenring, welcher als Anschlag 50 dient, gepresst wird. Auf diese Weise wird durch die Feder 40 ein spielfreier Sitz der Hülse 32 auf dem Schaft 14 gewährleistet. Um die Kupplung zu dem Arbeitsende zu lösen, wird die Hülse 32 gegen die Kraft der Feder 40 in proximaler Richtung verschoben, um die Nut 47 mit der Sicherungskugel 46 in axialer Richtung in Übereinstimmung zu bringen. Wird die Hülse 32 jedoch wieder losgelassen, wird sie durch die Kraft der Feder 40 gegen den Wellenring 50 gedrückt, so dass sie eine spielfreie axiale Fixierung der Hülse 32 an dem Instrument 10 ergibt.

Die Hülse 32 ist Teil einer Befestigungsvorrichtung 30, über welche eine Vorrichtung zur Erfassung der räumlichen Position 20 an dem chirurgischen Instrument 10 angeordnet wird. Die Befestigungsvorrichtung 30 weist einen Arm 31 auf, an dessen einem Ende die Hülse 32 angeordnet ist, während an dessen anderem Ende ein Adapter 34 angeordnet ist, an welchem die Vorrichtung zur Erfassung der räumlichen Position 20 angeordnet werden kann. Der Adapter 34 kann beispielsweise eine mechanische Rast-, Klemm- oder Schraubverbindung sein. Bei der Vorrichtung zur Erfassung der räumlichen Position 20 handelt es sich um einen sogenannten aktiven Tracker, welcher mehrere Infrarot-LEDs aufweist, die Infrarotlicht aussenden, welches von der Überwachungskamera, die in dem Operationssaal angeordnet ist, detektiert wird, so dass auf die räumliche Lage der Vorrichtung 20 im Operationssaal geschlossen werden kann.

Die Hülse 32 und somit die Befestigungsvorrichtung 30 und die Vorrichtung zur Erfassung der räumlichen Position 20 sind an dem Schaft 14 des chirurgischen Instruments 10 drehbar gelagert. Dazu sind zwischen dem Schaft 14 und der Innenseite der Hülse 32 ein erstes Kugellager 41 und ein zweites Kugellager 42 angeordnet. Die Kugeln der beiden Kugellager 41, 42 liegen in hohlkehlenförmigen umlaufenden Ausnehmungen 43, 44 in dem Schaft 14. Die Tiefe der Ausnehmungen 43, 44 ist dabei geringfügig geringer als der Durchmesser der Kugeln der Kugellager 41, 42, so dass die Kugeln geringfügig über die Außenfläche des Schafts 14 hervorragen und so einerseits eine radialer Fixierung und Stabilisierung der Hülse 32 auf dem Schaft 14 gewährleisten, andererseits jedoch eine geringe Kontaktfläche und somit eine geringe Reibung bei der Drehung ermöglichen. Da der Arm 31 der Befestigungsvorrichtung 30 starr an der Hülse 32 fixiert ist, dreht sich bei Drehung der Hülse 32 um den Schaft 14 die Vorrichtung 20 um den Schaft 14 des Instruments 10. Dabei kann der Schaft 14 des chirurgischen Instruments 10 beliebig in der Hülse 32 um seine Längsachse l₁₀ gedreht werden. Wird somit die Hülse 32 mit einer Hand festgehalten, kann während einer Operation das chirurgische Instrument 10 beliebig um seine Längsachse I₁₀ gedreht werden. Sofern vermieden wird, die Hülse 32 gegen die Kraft der Feder 40 zu bewegen, ist durch die Kraft der Feder 40 zudem eine spielfreie axiale Fixierung gewährleistet. Die Hülse 32 kann somit während der Operation immer derart gehalten werden, dass sich die Vorrichtung zur Erfassung der räumlichen Position 20 im Wesentlichen oberhalb des chirurgischen Instruments 10 befindet und somit für die in dem Operationsraum angeordnete Überwachungskamera grundsätzlich immer im Blickfeld liegt, unabhängig davon, wie stark das chirurgische Instrument 10 um seine Längsachse I₁₀ gedreht wird.

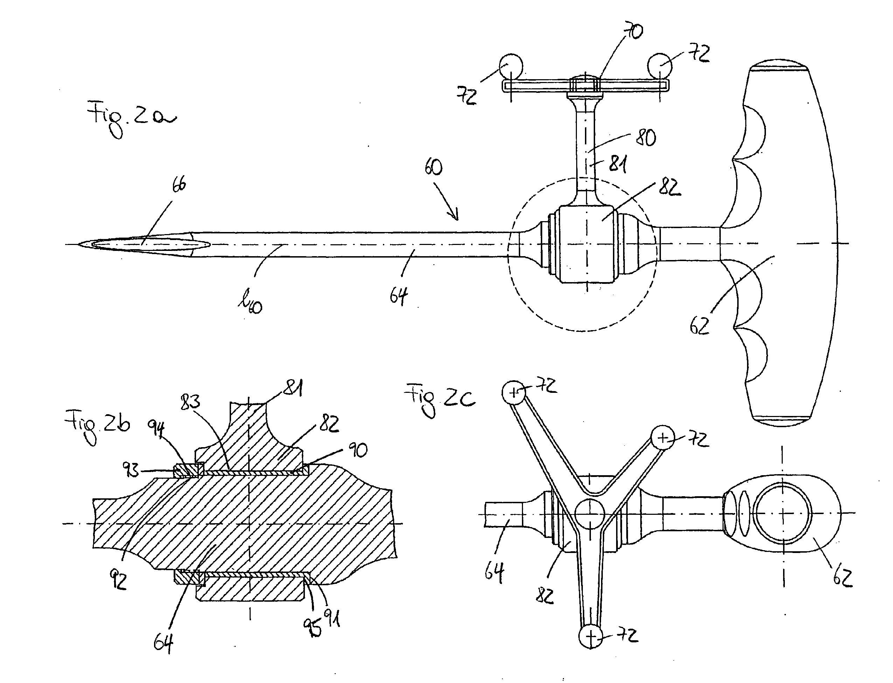

Ein weiteres Ausführungsbeispiel der Erfindung zeigen die Figuren 2a, 2b und 2c. Fig. 2a zeigt ein chirurgisches Instrument 60 mit einem Griff 62 und einem daran angeordneten Schaft 64. Die Längsachse des Schafts 64 definiert eine Längsachse l₆₀ des chirurgischen Instruments 60. Das distale Ende des Schafts 64 ist in diesem Ausführungsbeispiel nicht als Kupplung, sondern als Arbeitsende 66 ausgebildet. Bei dem Arbeitsende 66 handelt es sich um einen Dorn, welcher beispielsweise in einen Knochen eingetrieben werden muss. An dem Schaft 64 des Instruments 60 ist über eine Befestigungsvorrichtung 80 eine Vorrichtung zur Erfassung der räumlichen Position 70 angeordnet.

Bei der Vorrichtung zur Erfassung der räumlichen Position 70 handelt es sich um einen sogenannten passiven Tracker, welcher an den freien Enden eines etwa Y-förmigen Elements jeweils einen Reflektor 72 aufweist, an welchem Licht, welches von wenigstens einer, vorzugsweise von zwei in dem Operationssaal angeordneten Leuchtdioden reflektiert wird, welches anschließend in einer Kamera erfasst wird. Aus dem Bild der Signale der Reflektoren 72 kann ebenfalls auf die räumliche Lage der Vorrichtung zur Erfassung der räumlichen Position 70 geschlossen werden.

Die Befestigungsvorrichtung 80 weist einen Arm 81 auf, an deren einem Ende eine Hülse 82 angeordnet ist, während an dem anderen Ende des Arms 81 die Vorrichtung zur Erfassung der räumlichen Position 70 entweder unlösbar oder auswechselbar angeordnet ist. Die Hülse 82 weist eine Durchgangsöffnung 83 auf, welche von dem Schaft 64 des Instruments 60 durchsetzt wird. An der Innenseite der Hülse 82 liegt eine Lagerbuchse 90 an, welche ein Gleitlager zwischen der Befestigungsvorrichtung 80 und dem Schaft 64 bildet. An dem Schaft 64 ist ein Anschlag 91 angeordnet, gegen welchen die Lagerbuchse 90, wenn sie von distalen Ende des Schafts 64 auf den Schaft 64 aufgeschoben wird, anschlägt. Die Lagerbuchse 90 weist an ihrem proximalen Ende einen umlaufenden Vorsprung 95 auf, an welchem die Hülse 82, wenn sie von dem distalen Ende des Schafts 64 auf den Schaft 64 und die Lagerbuchse 90 aufgeschoben wird, anschlägt. Um die Hülse 82 in axialer Richtung fixieren zu können, wird nach dem Aufschieben der Hülse 82 auf den Schaft 64 eine Mutter 93 ausgehend vom distalen Ende des Schafts 64 auf den Schaft 64 aufgeschoben, welche mit einem Innengewinde 94 in ein an dem Schaft 64 angeordnetes Gewinde 92 eingreift, wobei bei Anziehen der Mutter 93 die Hülse 82 gegen den Vorsprung 95 der Lagerbuchse 90 und somit die Lagerbuchse 90 gegen den Anschlag 91 des Schafts 64 gepresst wird. Auf diese Weise wird eine spielfreie axiale Fixierung der Hülse 82 und damit insbesondere der Befestigungsvorrichtung 80 an dem Schaft 64 erreicht. Sobald die Mutter 93 wie gewünscht angezogen ist, kann die Mutter 93 in dieser Position fixiert werden, beispielsweise durch Laserverschweißen, Verkleben oder eine zusätzliche mechanische Arretiervorrichtung.

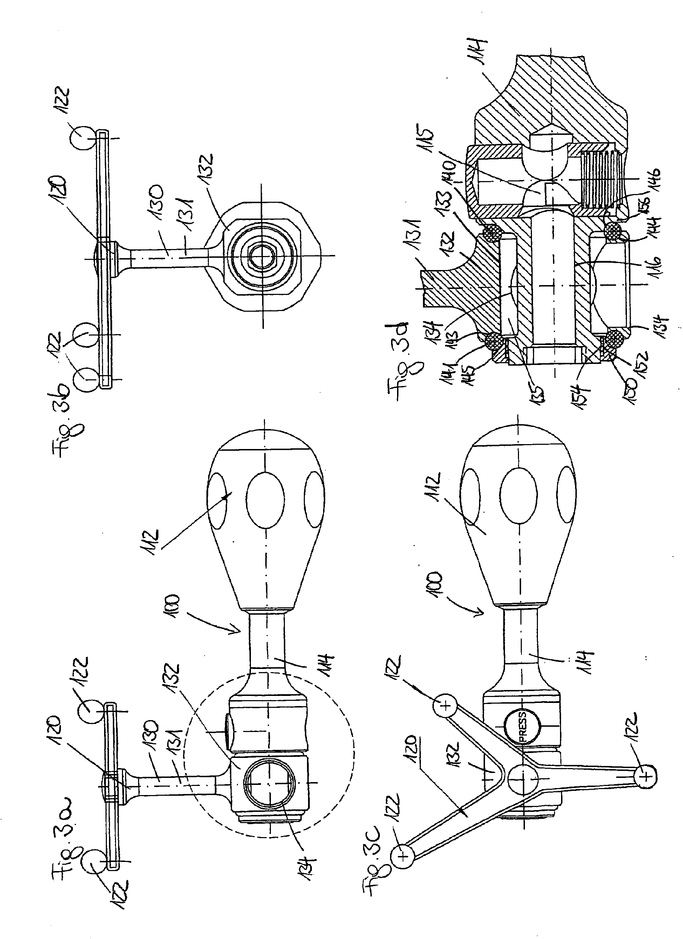

Ein weiteres Ausführungsbeispiel gemäß der Erfindung zeigen die Figuren 3a, 3b, 3c und 3d. Die Figuren 3a bis 3d zeigen ein chirurgisches Instrument 10 mit einem Griff 112, an welchem ein Schaft 114 angeordnet ist. Die Längsachse des Schafts 114 definiert eine Längsachse l₁₀₀ des chirurgischen Instruments 100.

Das distale Ende des Schafts 114 ist als Kupplung 115 ausgebildet, über welche ein Arbeitsende an dem Schaft 114 angeordnet werden kann. Dazu ist in dem distalen Ende des Schafts 114 eine Längsbohrung 116 angeordnet, in welche ein proximales Ende des Arbeitsendes eingesetzt und über die Kupplung 115 verriegelt werden kann.

Auf den Schaft 114 des Instruments 100 ist vom distalen Ende des Schafts 114 her eine Hülse 132 aufgeschoben, welche an einem Ende eines Arms 131 einer Befestigungsvorrichtung 130 angeordnet ist. Am anderen Ende des Arms 131 ist eine Vorrichtung zur Erfassung der räumlichen Position 120 angeordnet, welchen im vorliegenden Ausführungsbeispiel wiederum als passiver Tracker mit drei Reflektoren 122 ausgebildet ist, welche an den freien Enden eines im Wesentlichen Y-förmigen Elements angeordnet sind. Die Hülse 132 weist eine Durchgangsöffnung 133 auf, in welche der Schaft 115 des Instruments 100 eingesetzt ist. Wie insbesondere in Fig. 3a zu sehen ist, weist die Hülse 132 mehrere Wandöffnungen 134 auf, durch welche ein Innenraum 135, welcher zwischen Schaft 114 und der Innenwand der Hülse 132 liegt, zugänglich ist. Dies ermöglicht eine besonders einfache und gründliche Reinigung, wenn das Instrument 100 nach der Benutzung gereinigt und sterilisiert werden soll.

Die drehbare Lagerung der Hülse 132 auf dem Schaft 114 erfolgt durch zwei Schrägkugellager 140, 142, welche in O-Anordnung zueinander angeordnet sind. Das erste Schrägkugellager 140 ist zwischen dem proximalen Ende der Hülse 132 und dem Schaft 114 angeordnet. Dazu weist das proximale Ende der Hülse 132 eine Lauffläche 144 auf, deren Kontur im Schnitt im Wesentlichen einem Viertelkreis entspricht. Die Lauffläche 144 schneidet dazu die im Wesentlichen zylindrische Innenfläche der Hülse 132 und die Stirnfläche der Hülse 132 jeweils senkrecht. Dadurch verläuft die Lauffläche 144 ausgehend von der Innenwand der Hülse 132 zunächst im Wesentlichen senkrecht zur Längsachse l₁₀₀ des Instrumentes 100 und krümmt sich derart, dass sie an der Stirnseite der Hülse 132 im Wesentlichen parallel zur Längsachse l₁₀₀ verläuft. Die entsprechende mit der Lauffläche 144 zusammenwirkende Lauffläche 146 weist ebenfalls im Schnitt im Wesentlichen die Form eines Viertelkreises auf, wobei diese Lauffläche 144 zunächst parallel zur Außenwandung des Schafts 114 und somit parallel zur Längsachse I₁₀₀ des Instruments 100 verläuft, sich in proximaler Richtung anschließend derart nach außen krümmt, dass sie im Wesentlichen senkrecht zur Längsachse l₁₀₀ des Instruments 100 verläuft. Die Lauffläche 146 ist dabei über den Viertelkreis tangential nach außen gezogen und bildet damit einen Anschlag 156.

Das zweite Schrägkugellager 142 ist zwischen der distalen Stirnseite der Hülse 132 und einer Mutter 150 angeordnet. In der distalen Stirnseite der Hülse 132 ist dazu eine Lauffläche 143 angeordnet, welche im Wesentlichen symmetrisch zur Lauffläche 144 in der proximalen Stirnseite der Hülse 132 ausgebildet ist. Die Mutter 150 ist im Wesentlichen zylindrisch ausgebildet und weist ein Innengewinde 152 auf, welches in ein an dem Schaft 114 des Instruments 100 angeordnetes Gewinde 154 eingreift. Dabei wird zunächst die Hülse 132 auf den Schaft 114 ausgehend von dem distalen Ende des Schafts 114 aufgeschoben, woraufhin anschließend die Mutter 150 ebenfalls ausgehend vom distalen Ende des Schafts 114 auf den Schaft 114 aufgeschoben wird. Zur Bildung des Schrägkugellagers 142 weist die Mutter 150 in ihrer proximalen Stirnseite eine Lauffläche 145 auf, welche sowohl die proximale Stirnseite der Mutter 150 als auch die im wesentlichen zylindrische Außenwandung der Mutter 150 senkrecht schneidet, so dass die Lauffläche 145 an der proximalen Stirnseite der Mutter 150 im Wesentlichen parallel zur Längsachse l₁₀₀ des Instruments 100 verläuft, während sie sich anschließend in distaler Richtung derart krümmt, dass sie im Bereich der Außenwandung der Mutter 150 im Wesentlichen senkrecht zur Längsachse l₁₀₀ verläuft. Durch diese Ausbildung der Laufflächen 143, 144, 145, 146 der Schrägkugellager 140, 142 ist es möglich, die Hülse 132 und somit die Befestigungsvorrichtung 130 spielfrei sowohl in axialer als auch in radialer Richtung zu fixieren, wenn die Mutter 150 auf dem Gewinde 154 angezogen wird, so dass die Befestigungsvorrichtung 130 in proximaler Richtung gegen den Anschlag 156 gepresst wird.

Ist die Mutter 150 in die gewünschte Position angezogen, wird sie bevorzugt in dieser Position fixiert, beispielsweise mittels Laserschweißen, Verkleben oder einer sonstigen mechanischen Arretiervorrichtung.

Dadurch, dass die Schrägkugellager 140, 142 von außen weiterhin zugänglich sind und die Hülse 132 durch die Wandöffnungen 134 durchgebrochen ist, ist auch der Innenraum 135 zwischen der Hülse 132 und dem Schaft 114 besonders einfach zu reinigen.

Es ist selbstverständlich, dass die verschiedenen in den Figuren 1b, 2b, 3b dargestellten drehbaren Lagerungen der Befestigungsvorrichtungen 30, 80, 130 beliebig mit den verschiedenen Ausgestaltungen der Vorrichtung zur Erfassung der räumlichen Position, nämlich dem aktiven Tracker 20 oder dem passiven Tracker 70, 120, und den verschiedenen chirurgischen Instrumenten 10, 60, 100, nämlich sowohl Instrumenten mit verschiedenen Arten von Kupplungen als auch Instrumenten mit fest daran angeordnetem Arbeitsende 66, kombiniert werden können.

### Bezugszeichenliste

- 10: Instrument
- 12: Griff
- 14: Schaft
- 20: Vorrichtung
- 30: Befestigungsvorrichtung
- 31: Arm
- 32: Hülse
- 33: Durchgangsöffnung
- 34: Adapter
- 40: Feder
- 41: Kugellager
- 42: Kugellager
- 43: Ausnehmung
- 44: Ausnehmung
- 45: Längsbohrung
- 46: Sicherungskugel
- 47: Nut
- 48: Vorsprung
- 49: Vorsprung
- 50: Anschlag
- l₁₀: Längsachse

- 60: Instrument
- 62: Griff
- 64: Schaft
- 66: Arbeitsende
- 70: Vorrichtung
- 72: Reflektor
- 80: Befestigungsvorrichtung
- 81: Arm
- 82: Hülse
- 83: Durchgangsöffnung
- 90: Lagerbuchse
- 91: Anschlag
- 92: Gewinde
- 93: Mutter
- 94: Innengewinde
- 95: Vorsprung
- l₆₀: Längsachse

- 100: Instrument
- 112: Griff
- 114: Schaft
- 115: Kupplung
- 116: Längsbohrung
- 120: Vorrichtung
- 122: Reflektor
- 130: Befestigungsvorrichtung
- 131: Arm
- 132: Hülse
- 133: Durchgangsöffnung
- 134: Wandöffnung
- 135: Innenraum
- 140: Schrägkugellager
- 142: Schrägkugellager
- 143: Lauffläche
- 144: Lauffläche
- 145: Lauffläche
- 146: Lauffläche
- 150: Mutter
- 152: Innengewinde
- 154: Gewinde
- 156: Anschlag
- l₁₀₀: Längsachse

## Patentansprüche

1. Vorrichtung (20, 70, 120) zur Erfassung der räumlichen Position, welche über eine Befestigungsvorrichtung (30, 80, 130) an einem chirurgischen Instrument (10, 60, 100), welches eine Längsachse (l₁₀, l₆₀, l₁₀₀) aufweist, anbringbar ist,
**dadurch gekennzeichnet, dass** die Befestigungsvorrichtung (30, 80, 130) eine Durchgangsöffnung (33, 83, 133) aufweist, welche von dem Instrument (10, 60, 100) durchsetzt wird, und dass die Befestigungsvorrichtung (30, 80, 130) um die Längsachse (l₁₀, l₆₀, l₁₀₀) des Instruments (10, 60, 100) drehbar gelagert ist.

2. Vorrichtung nach Anspruch 1,
**dadurch gekennzeichnet, dass** die Befestigungsvorrichtung (30, 80, 130) in axialer und/oder radialer Richtung fixierbar ist.

3. Vorrichtung nach Anspruch 2,
**dadurch gekennzeichnet, dass** zur axialen und/oder radialen Fixierung eine umlaufende Nut und/oder ein umlaufender Vorsprung und/oder ein umlaufender Anschlag (50, 91, 156) vorgesehen sind.

4. Vorrichtung nach Anspruch 2 oder 3,
**dadurch gekennzeichnet, dass** zur axialen und/oder radialen Fixierung wenigstens eine in der Durchgangsöffnung (83) angeordnete Lagerbuchse (90), insbesondere eine Kunststofflagerbuchse, vorgesehen ist.

5. Vorrichtung nach einem der Ansprüche 2 bis 4,
**dadurch gekennzeichnet, dass** zur axialen und/oder radialen Fixierung ein Kugellager (41, 42, 140, 142) vorgesehen ist.

6. Vorrichtung nach einem der Ansprüche 2 bis 5,
**dadurch gekennzeichnet, dass** zur axialen und radialen Fixierung zwei Schrägkugellager (140, 142) vorgesehen sind.

7. Vorrichtung nach einem der Ansprüche 2 bis 6,
**dadurch gekennzeichnet, dass** zur axialen Fixierung eine Feder (40) vorgesehen ist, welche die Befestigungsvorrichtung (30) gegen einen an dem Instrument angeordneten Anschlag (50) presst.

8. Vorrichtung nach einem der Ansprüche 2 bis 6,
**dadurch gekennzeichnet, dass** zur axialen Fixierung eine Mutter (93, 150) mit einem Gewinde (94, 152) vorgesehen ist, welche in ein an dem Instrument (60, 100) angeordnetes Gewinde (92, 154) eingreift, wobei ein Anziehen der Mutter (93, 150) die Befestigungsvorrichtung (80, 130) gegen einen an dem Instrument (60, 100) angeordneten Anschlag (91, 156) presst.

9. Vorrichtung nach einem Anspruch 8,
**dadurch gekennzeichnet, dass** die Mutter (93, 150) in der gewünschten Position fixierbar ist.

10. Vorrichtung nach einem der vorhergehenden Ansprüche,
**dadurch gekennzeichnet, dass** die Vorrichtung zur Erfassung der räumlichen Position (20, 70, 120) als aktiver oder passiver Tracker ausgebildet ist.

11. Vorrichtung nach einem der vorhergehenden Ansprüche,
**dadurch gekennzeichnet, dass** die Vorrichtung als optischer Tracker ausgebildet ist oder ein GPS-System aufweist.

## Geänderte Patentansprüche

### Geänderte Patentansprüche gemäss Regel 137(2) EPÜ.

**1.** Vorrichtung (120) zur Erfassung der räumlichen Position, welche über eine Befestigungsvorrichtung (130) an einem chirurgischen Instrument (100), welches eine Längsachse (l₁₀₀) aufweist, anbringbar ist, wobei die Befestigungsvorrichtung (130) eine Durchgangsöffnung (133) aufweist, welche von dem Instrument (100) durchsetzt wird, und wobei die Befestigungsvorrichtung (130) um die Längsachse (l₁₀₀) des Instruments (100) drehbar gelagert ist,
**dadurch gekennzeichnet, dass** die Befestigungsvorrichtung (130) in axialer und radialer Richtung mittels zweier Schrägkugellager (140, 142) fixierbar ist.

**2.** Vorrichtung nach Anspruch 1,
**dadurch gekennzeichnet, dass** zur axialen und/oder radialen Fixierung eine umlaufende Nut und/oder ein umlaufender Vorsprung und/oder ein umlaufender Anschlag (156) vorgesehen sind.

**3.** Vorrichtung nach Anspruche 1 oder 2,
**dadurch gekennzeichnet, dass** zur axialen Fixierung eine Mutter (150) mit einem Gewinde (152) vorgesehen ist, welche in ein an dem Instrument (100) angeordnetes Gewinde (154) eingreift, wobei ein Anziehen der Mutter (150) die Befestigungsvorrichtung (130) gegen einen an dem Instrument (100) angeordneten Anschlag (156) presst.

**4.** Vorrichtung nach einem Anspruch 3,
**dadurch gekennzeichnet, dass** die Mutter (150) in der gewünschten Position fixierbar ist.

**5.** Vorrichtung nach einem der vorhergehenden Ansprüche,
**dadurch gekennzeichnet, dass** die Vorrichtung zur Erfassung der räumlichen Position (120) als aktiver oder passiver Tracker ausgebildet ist.

**6.** Vorrichtung nach einem der vorhergehenden Ansprüche,
**dadurch gekennzeichnet, dass** die Vorrichtung als optischer Tracker ausgebildet ist oder ein GPS-System aufweist.
